# EUROPÄISCHE PATENTSCHRIFT

(11) **EP 1 381 628 B1**
(45) Veröffentlichungstag und Bekanntmachung des Hinweises auf die Patenterteilung: **23.11.2005**
(21) Anmeldenummer: 02747282.8
(22) Anmeldetag: 16.04.2002
(51) Int. Cl.: C07K 14/47, G01N 33/68

(54) **ENTZÜNDUNGSSPEZIFISCHE PEPTIDE UND DEREN VERWENDUNGEN**
INFLAMMATION-SPECIFIC PEPTIDES AND THE USES THEREOF
PEPTIDES SPECIFIQUES D'INFLAMMATIONS ET LEURS UTILISATIONS

(30) Priorität: 23.04.2001 DE 10119804
(43) Veröffentlichungstag der Anmeldung: 21.01.2004
(73) Patentinhaber: B.R.A.H.M.S Aktiengesellschaft, 16761 Hennigsdorf (DE)
(72) Erfinder: BERGMANN, Andreas, 12351 Berlin (DE); STRUCK, Joachim, 12161 Berlin (DE); ÜHLEIN, Monika, 10407 Berlin (DE)
(74) Vertreter: Andrae, Steffen
(86) Internationale Anmeldenummer: PCT/EP2002/004219
(87) Internationale Veröffentlichungsnummer: WO 2002/085937

(56) Entgegenhaltungen:
- WO-A-98/11217
- WO-A-99/47669
- DATABASE EMBL [Online] LAMERDIN ET AL.: "R33729_1, partial CDS (Fragment)" Database accession no. O75272 XP002213090
- REINHART K ET AL: "SEPSIS UND SPETISCHER SCHOCK" INTENSIVMEDIZIN, XX, XX, 2001, Seiten 756-760, XP008003130 in der Anmeldung erwähnt

## Beschreibung

Die vorliegende Erfindung betrifft eine neue Verwendung von bestimmten Peptiden, wie sie in der nachfolgenden Beschreibung näher erläutert werden, im Rahmen der medizinischen *in vitro* Diagnostik von Entzündungen und Infektionen, insbesondere im Zusammenhang mit Sepsis.

Der Begriff "Peptide" wird dabei in der vorliegenden Anmeldung im Sinne eines Oberbegriffs verwendet, der Kondensationsprodukte von Aminosäuren unabhängig von der Länge der gebildeten Kette umfassen soll, also insbesondere Produkte, die unter Berücksichtigung ihrer Kettenlänge als Öligopeptide, Polypeptide oder Proteine bezeichnet werden können.

Die vorliegende Erfindung hat ihren Ursprung in intensiven Forschungsarbeiten der Anmelderin im Zusammenhang mit weiteren Verbesserungen der Diagnose und Therapie von Entzündungen und Infektionen, insbesondere von Entzündungen infektiöser Ätiologie und Sepsis.

Als Entzündungen (Inflammationen) werden ganz allgemein bestimmte physiologische Reaktionen eines Organismus auf verschiedenartige äußere Einwirkungen wie z.B. Verletzungen, Verbrennungen, Allergene, Infektionen durch Mikroorganismen wie Bakterien und Pilze und Viren, auf Fremdgewebe, die Abstoßungsreaktionen auslösen, oder auf bestimmte entzündungsauslösende endogene Zustände des Körpers, z.B. bei Autoimmunerkrankungen und Krebs, bezeichnet. Entzündungen können als harmlose, lokal begrenzte Reaktionen des Körpers auftreten, sind jedoch auch typische Merkmale zahlreicher ernster chronischer und akuter Erkrankungen von einzelnen Geweben, Organen, Organ- und Gewebsteilen.

Lokale Entzündungen sind dabei meist Teil der gesunden Immunreaktion des Körpers auf schädliche Einwirkungen, und damit Teil des lebenserhaltenden Abwehrmechanismus des Organismus. Wenn Entzündungen jedoch Teil einer fehlgeleiteten Reaktion des Körpers auf bestimmte endogene Vorgänge wie z.B. bei Autoimmunerkrankungen sind und/oder chronischer Natur sind, oder wenn sie systemische Ausmaße erreichen, wie beim systemischen Inflammationssyndrom (Systemic Inflammatory Response Syndrome, SIRS) oder bei einer auf infektiöse Ursachen zurückzuführenden schweren Sepsis, geraten die für Entzündungsreaktionen typischen physiologischen Vorgänge außer Kontrolle und werden zum eigentlichen, häufig lebensbedrohlichen Krankheitsgeschehen.

Es ist heute bekannt, dass die Entstehung und der Verlauf von entzündlichen Prozessen von einer beträchtlichen Anzahl von Substanzen, die überwiegend proteinischer bzw. peptidischer Natur sind, gesteuert werden bzw. von einem mehr oder weniger zeitlich begrenzten Auftreten bestimmter Biomoleküle begleitet sind. Zu den an Entzündungsreaktionen beteiligten endogenen Substanzen gehören insbesondere solche, die zu den Cytokinen, Mediatoren, vasoaktiven Substanzen, Akutphasenproteinen und/oder hormonellen Regulatoren gezählt werden können. Die Entzündungsreaktion stellt eine komplexe physiologische Reaktion dar, an der sowohl das Entzündungsgeschehen aktivierende endogene Substanzen (z.B. TNF-α) als auch desaktivierende Substanzen (z.B. Interleukin-10) beteiligt sind.

Bei systemischen Entzündungen wie im Falle einer Sepsis bzw. des septischen Schocks weiten sich die entzündungsspezifischen Reaktionskaskaden unkontrolliert auf den gesamten Körper aus und werden dabei, im Sinne einer überschießenden Immunantwort, lebensbedrohlich. Zu den gegenwärtigen Kenntnissen über das Auftreten und die möglichen Rolle einzelner Gruppen endogener entzündungsspezifischer Substanzen wird beispielsweise verwiesen auf A.Beishuizen, et al., "Endogenous Mediators in Sepsis and Septic Shock", Advances in Clinical Chemistry, Vol.33, 1999, 55-131; und C.Gabay, et al., "Acute Phase Proteins and Other Systemic Responses to Inflammation", The New England Journal of Medicine, Vol.340, No.6, 1999, 448-454. Da sich das Verständnis von Sepsis und verwandten systemischen entzündlichen Erkrankungen, und damit auch die anerkannten Definitionen, in den letzten Jahren gewandelt haben, wird außerdem verwiesen auf K.Reinhart, et al., "Sepsis und septischer Schock", in: Intensivmedizin, Georg Thieme Verlag, Stuttgart.New York, 2001, 756-760; wo eine moderne Definition des Sepsis-Begriffes gegeben wird. Im Rahmen der vorliegenden Anmeldung werden die Begriffe Sepsis bzw. entzündliche Erkrankungen in Anlehnung an die Definitionen verwendet, wie sie den genannten drei Literaturstellen entnommen werden können.

Während wenigstens im europäischen Raum die durch eine positive Blutkultur nachweisbare systemische bakterielle Infektion lange den Sepsisbegriff prägte, wird die Sepsis heute in erster Linie als systemische Entzündung verstanden, die infektiöse Ursachen hat, als Krankheitsgeschehen jedoch große Ähnlichkeiten mit systemischen Entzündungen aufweist, die durch andere Ursachen ausgelöst werden. Dem genannten Wandel des Sepsis-Verständnisses entsprechen Veränderungen der diagnostischen Ansätze. So wurde der direkte Nachweis bakterieller Erreger durch komplexe Überwachungen physiologischer Parameter und in jüngerer Zeit insbesondere auch durch den Nachweis bestimmter am Sepsisgeschehen bzw. am Entzündungsgeschehen beteiligter endogener Substanzen, d.h. spezifischer "Biomarker", ersetzt bzw. ergänzt.

Von der großen Zahl von Mediatoren und Akutphasenproteinen, von denen man weiß, dass sie an einem Entzündungsgeschehen beteiligt sind, eignen sich dabei für diagnostische Zwecke insbesondere solche, deren Auftreten sehr spezifisch für entzündliche Erkrankungen bzw. bestimmte Phasen entzündlicher Erkrankungen ist, deren Konzentrationen sich drastisch und diagnostisch signifikant verändern und die außerdem die für Routinebestimmungen erforderlichen Stabilitäten aufweisen und hohe Konzentrationswerte erreichen. Für diagnostische Zwecke steht dabei die zuverlässige Korrelation von Krankheitsgeschehen (Entzündung, Sepsis) mit dem jeweiligen Biomarker im Vordergrund, ohne dass dessen Rolle in der komplexen Kaskade der am Entzündungsgeschehen beteiligten endogenen Substanzen bekannt sein muss.

Eine derartige als Sepsis-Biomarker besonders geeignete endogene Substanz ist Procalcitonin. Procalcitonin ist ein Prohormon, dessen Serum-Konzentrationen unter den Bedingungen einer systemischen Entzündung infektiöser Ätiologie (Sepsis) sehr hohe Werte erreichen, während es bei Gesunden so gut wie nicht nachweisbar ist. Hohe Werte an Procalcitonin werden außerdem in einem relativ frühen Stadium einer Sepsis erreicht, so dass sich die Bestimmung von Procalcitonin auch zu Früherkennung einer Sepsis und zur frühen Unterscheidung einer infektiös bedingten Sepsis von schweren Entzündungen, die auf anderen Ursachen beruhen, eignet. Die Bestimmung von Procalcitonin als Sepsismarker ist Gegenstand der Veröffentlichung M. Assicot, et al., "High serum procalcitonin concentrations in patients with sepsis and infection", The Lancet, vol.341, No.8844, 1993, 515-518; und der Patente DE 42 27 454 C2 bzw. EP 0 656 121 B1 bzw. US 5,639,617. Auf die genannten Patente und in der genannten Veröffentlichung angeführten frühen Literaturstellen wird zur Ergänzung der vorliegenden Beschreibung ausdrücklich Bezug genommen. In den letzten Jahren ist die Zahl der Veröffentlichungen zum Thema Procalcitonin stark angestiegen. Stellvertretend für zusammenfassende Veröffentlichungen aus jüngerer Zeit wird daher noch verwiesen auf W.Karzai et al., "Procalcitonin - A New Indicator of the Systemic Response to Severe infection", Infection, Vol. 25, 1997, 329-334; und M.Oczenski et al., "Procalcitonin: a new parameter for the diagnosis of bacterial infection in the peri-operative period", European Journal of Anaesthesiology 1998, 15, 202-209; sowie ferner H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", in: Sepsis 1998; 2:243-253; und die darin zitierten weiteren Literaturstellen.

Die Verfügbarkeit des Sepsismarkers Procalcitonin hat der Sepsisforschung starke Impulse gegeben, und es werden gegenwärtig intensive Anstrengungen unternommen, weitere Biomarker zu finden, die die Procalcitonin-Bestimmung ergänzen können und/oder zusätzliche Informationen für Zwecke der Feindiagnostik bzw. Differentialdiagnostik zu liefern vermögen. Erschwert wird die Suche nach potentiellen neuen Sepsis-Biomarkern allerdings dadurch, dass häufig noch sehr wenig oder nichts über die genaue Funktion bzw. über die genauen Gründe für das Auftreten bestimmter endogener Substanzen, die am Entzündungs- oder Sepsisgeschehen beteiligt sind, bekannt ist.

Die Ergebnisse der experimentellen Überprüfung eines fruchtbaren rein hypothetischen Ansatzes zur Ermittlung weiterer potentieller Sepsismarker finden sich in DE 198 47 690 A1 bzw. WO 00/22439. Dort wird gezeigt, dass bei Sepsis nicht nur die Konzentration des Prohormons Procalcitonin erhöht ist, sondern auch für andere Substanzen, die zu den Peptid-Prohormonen gerechnet werden können, signifikant erhöhte Konzentrationen beobachtet werden können. Während das beschriebene Phänomen gut dokumentiert ist, sind die Ursachen für den Anstieg der Konzentrationen von Prohormonen bei Sepsis weiterhin weitgehend ungeklärt.

In der vorliegenden Anmeldung wird nunmehr ein erstes Ergebnis eines anderen, rein experimentellen Ansatzes für die Suche nach weiteren entzündungs- bzw. sepsisspezifischen Biomolekülen berichtet. Auch diese experimentellen Untersuchungen nehmen ihren Ausgang bei der Bestimmung von Procalcitin im Zusammenhang mit systemischen entzündlichen Reaktionen infektiöser Ätiologie. So war sehr früh beobachtet worden, dass bei Sepsis das Procalcitonin offensichtlich nicht auf die gleiche Weise gebildet wird, wie dann, wenn es Vorläufer für das Hormon Calcitonin ist. So wurden hohe Procalcitoninspiegel auch bei Patienten beobachtet, denen die Schilddrüse entfernt worden war. Deshalb kann die Schilddrüse nicht dasjenige Organ sein, in dem Procalcitonin bei Sepsis gebildet bzw. ausgeschüttet wird. In den Veröffentlichungen H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", Sepsis 1998; 2:243-253; werden die Ergebnissen von experimentellen Untersuchungen berichtet, die der Klärung der Bildung von Procalcitonin bei Sepsis dienen sollten. In den genannten Arbeiten wird durch Endotoxinverabreichung an Primaten (Paviane) eine künstliche Sepsis erzeugt, und es wird bestimmt, bei welchen experimentell erzeugten Zuständen die höchsten Procalcitoninkonzentrationen im Blut erreicht werden. Eine Weiterentwicklung des in den genannten Arbeiten beschriebene Versuchstiermodells dient im Rahmen der vorliegenden Anmeldung dazu, neue endogene sepsisspezifische Biomarker von peptischer bzw. proteinischer Natur zu ermitteln, deren Auftreten für Sepsis oder bestimmte Formen von Sepsis charakteristisch ist und die daher eine spezifische Sepsisdiagnose ermöglichen. Das Primatenmodell wurde dabei aufgrund der sehr großen Ähnlichkeit der Physiologie von Primaten und Menschen und der hohen Kreuzreaktivität mit vielen therapeutischen und diagnostischen humanen Reagenzien gewählt.

Da die bei Entzündungen gebildeten endogenen Substanzen Teil der komplexen Reaktionskaskade des Körpers sind, sind derartige Substanzen außerdem nicht nur von diagnostischem Interesse, sondern es wird gegenwärtig auch mit erheblichem Aufwand versucht, durch Beeinflussung der Entstehung und/oder der Konzentration einzelner derartiger Substanzen therapeutisch in das Entzündungsgeschehen einzugreifen, um die zum Beispiel bei Sepsis beobachtete systemische Ausbreitung der Entzündung möglichst frühzeitig zu stoppen. In diesem Sinne sind nachweislich am Entzündungsgeschehen beteiligte endogene Substanzen auch als potentielle therapeutische Targets anzusehen. Versuche, ansetzend an bestimmten Mediatoren des Entzündungsgeschehens dieses positiv therapeutisch zu beeinflussen, sind beschrieben beispielsweise in E.A.Panacek, "Anti-TNF strategies", Journal für Anästhesie und Intensivbehandlung; Nr.2, 2001, 4-5; T.Calandra, et al., "Protection from septic shock by neutralization of macrophage migration inhibitory factor", Nature Medicine, Vol.6, No.2, 2000, 164-170; oder K.Garber, "Protein C may be sepsis solution", Nature Biotechnology, Vol.18, 2000, 917-918. Diese therapeutischen Ansätze laufen darauf hinaus, die Konzentrationen entzündungsfördernder Substanzen zu senken bzw. die Entstehung derartiger Substanzen zu hemmen, und zwar insbesondere unter Verwendung von spezifischen Antikörpern (gegen TNF-α bzw. MIF; vgl. E.A. Panacek, "Anti-TNF strategies", Journal für Anästhesie und Intensivbehandlung; Nr.2, 2001, 4-5; T.Calandra, et al., "Protection from septic shock by neutralization of macrophage migration inhibitory factor", Nature Medicine, Vol.6, No.2, 2000, 164-170) bzw. die Konzentration von hemmend in die Entzündungskaskade eingreifenden endogenen Substanzen (Protein C; K.Garber, "Protein C may be sepsis solution", Nature Biotechnology, Vol.18, 2000, 917-918) zu erhöhen. In der letztgenannten Veröffentlichung findet sich ein Überblick über derartige, leider bisher meist wenig erfolgreiche Versuche, das Entzündungsgeschehen unter Beeinflussung ausgewählter endogener Target-Moleküle therapeutisch zu beeinflussen. Angesichts der bisherigen eher enttäuschenden therapeutischen Ansätze besteht ein hohes Interesse daran, weitere möglichst entzündungs- bzw. sepsisspezifische endogene Biomoleküle zu identifizieren, die auch als therapeutische Targets neue Erfolgsaussichten für die Entzündungsbekämpfung eröffnen.

Gemäß der vorliegenden Erfindung werden Peptide angegeben, die in Primaten und Menschen bei infektiös bedingten Entzündungen gebildet werden und für die erstmals festgestellt wurde, dass sie als Markerpeptide für die Entzündungsdiagnostik/Sepsisdiagnostik geeignet sind.

Demgemäß betrifft die vorliegende Erfindung gemäß Anspruch 1 die Verwendung eines Peptids gemäß SEQ ID NO:4 oder SEQ ID NO:5 als Markerpeptid in in vitro Verfahren zum diagnostischen Nachweis und zur Verlaufskontrolle von Entzündungen und Infektionen.

Bevorzugte Möglichkeiten einer solchen Verwendung sind in den Ansprüchen 2 bis 4 angegeben.

Wie nachfolgend im experimentellen Teil noch näher ausgeführt wird, beruht die Erfindung darauf, dass nach experimenteller Auslösung einer künstlichen Sepsis in Pavianen durch Endotoxinverabreichung (LPS aus Salmonella Typhimurium) und 2D-gelektrophoretischer Aufarbeitung von Lebergewebe der behandelten Tiere ein nur bei den behandelten Tieren identifizierbares Peptid- bzw. Proteinprodukt gefunden werden konnte. Dieses spezifische Produkt wurde aus dem Elektrophoresegel isoliert und auf an sich bekannte Weise massenspektrometisch untersucht. Eine Teilsequenz aus 12 Aminosäuren (SEQ ID NO:1) konnte eindeutig identifiziert werden, und durch Vergleich der Sequenz dieses Teilpeptids mit den Daten einer redundanten humanen Datenbank mit Proteinsequenzen konnte die Sequenz als Teil von nur zwei aller in der Datenbank gespeicherten Peptidsequenzen identifiziert werden. Beide gehören offensichtlich zu dem gleichen hypothetischen Protein (R33729_1), für das die zugehörige Genom-DNA nach den verfügbaren Datenbank-Angaben auf Chromosom 19 lokalisiert ist. WO 98/11217 A1 beschreibt die Ergebnisse von Klonierversuchen von cDNAs mit sekretorischen Signalsequenzen, wobei unter den identifizierten Klonen ein als HP10029 bezeichneter Klon aus einem Epidermiscarcinom war, der für ein sich davon ableitendes Peptid aus 173 Aminosäureresten (gezeigt in Sequenz No.: 5 bzw. 23) kodiert, das die obige Sequenz gemäß SEQ ID NO:1 als Teilsequenz in den Positionen 32 bis 43 bzw. eine mit der Sequenz SEQ ID NO:4 der vorliegenden Anmeldung identische Sequenz zusammen mit einer Signalsequenz enthält.

Die eindeutige Identifizierung des bisher nur hypothetischen Proteins gemäß R33729_1 als Sepsisprotein ist von hohem wissenschaftlichen, diagnostischen und therapeutischen Interesse. Die Anmelderin prägte für dieses Protein die Bezeichnung "Präinflammin" und schlägt diesen Begriff als Bezeichnung für die zukünftige Benennung des genannten Proteins vor. Das im Rahmen der nachfolgend beschrieben gelelektrophoretischen Aufarbeitung direkt gefundene kürzere Peptid mit einer geringeren Molmasse, das vermutlich als das eigentliche Markerpeptid anzusehen ist, wird entsprechend als "Inflammin" bezeichnet.

Mit der vorliegenden Erfindung wird Schutz für die Verwendung eines solchen Peptids als Markerpeptid in in vitro Verfahren zum diagnostischen Nachweis und zur Verlaufskontrolle und Infektionen bzw. Sepsis beansprucht.

Als Teilsequenzen im Sinne der Verwendung dieses Begriffs in der vorliegenden Anmeldungen sind auch solche Sequenzen anzusehen, die nach Deletion von einer oder mehreren Aminosäuren oder kurzer Peptidsequenzen, z.B. eines Pentapeptids entsprechend den Positionen 59 bis 63 aus dem gesamten Peptid SEQ ID NO:5, oder aus dem Peptid SEQ ID NO:4 erhalten werden. Ferner sind für diagnostische und/oder therapeutische Zwecke geeignete Teilsequenzen (Fragmente) insbesondere solche, die eine Folge von mindestens drei Aminosäuren, vorzugsweise mindestens 6 Aminosäuren, des Peptids SEQ ID NO:4 umfassen bzw. entsprechende Teile der Aminosäuresequenz zwischen den Positionen 32 bis 178 von SEQ ID NO:5. Teilsequenzen im Sinne der Verwendung dieses Begriffs in der vorliegenden Beschreibungen können auch als Teile von Peptiden vorliegen, die durch Austausch und/oder Deletion einzelner Aminosäuren oder Peptidfragmente der Peptide gemäß SEQ ID NO:1, SEQ ID NO:4 und SEQ ID NO:5 gebildet werden.

Aufgrund der nunmehr bekannten Sequenz und der physiologischen Rolle der genannten Peptide können diese für diagnostische Zwecke nach Verfahren, die inzwischen zum Stand der Technik gehören, synthetisch oder gentechnologisch als Rekombinationsprodukte hergestellt werden.

Ferner können die genannten Peptide bzw. geeignete Teilsequenzen davon nach bekannten Verfahren des modernen Standes der Technik auch zur Erzeugung spezifischer polyklonaler und insbesondere monoklonaler Antikörper verwendet werden, die als Hilfsmittel für die diagnostische Bestimmung der erfindungsgemäßen Peptide und/oder auch als potentielle therapeutische Mittel,geeignet sind. Die Erzeugung geeigneter monoklonaler Antikörper gegen bekannte Peptid-Teilsequenzen gehört heute zum allgemeinen Stand der Technik.

Bei der Bestimmung von Inflammin oder von ausgewählten Teilpeptiden davon kann dabei grundsätzlich so vorgegangen werden, wie das z.B. für die selektive Procalcitoninbestimmung beschrieben ist in P.P.Ghillani, et al., "Monoclonal antipeptide antibodies as tools to dissect closely related gene products", The Journal of Immunology, vol. 141, No.9, 1988, 3156-3163; und P.P.Ghillani, et al., "Identification and Measurement of Calcitonin Precursors in Serum of Patients with Malignant Diseases", Cancer research, vol.49, No.23, 1989, 6845-6851; wobei ausdrücklich ergänzend auch auf die dort beschriebenen Immunisierungstechniken verwiesen wird, die eine Möglichkeit für die Gewinnung von monoklonalen Antikörpern auch gegen Teilsequenzen des Inflammins darstellen. Variationen der beschriebenen Techniken und/oder weitere Immunisierungstechniken kann der Fachmann einschlägigen Standardwerken und Veröffentlichungen entnehmen und sinngemäß anwenden.

Ferner ist auch ausdrücklich die Antikörpererzeugung unter Anwendung von Techniken der direkten genetischen Immunisierung mit DNA zu erwähnen. Es liegt ferner im Rahmen der vorliegenden Erfindung, zur Immunisierung z.B. eine cDNA der gewünschten Inflammin-Peptide zu verwenden, da es sich in der Vergangenheit gezeigt hat, dass durch derartige Immunisierungstechniken das Spektrum der gewinnbaren Antikörper erweitert werden kann. Inflammin gemäß SEQ ID NO:4 oder Teilpeptide davon, z.B. solche, die die Teilsequenz SEQ ID NO:1 und/oder andere Teilsequenzen enthalten, können aufgrund der vorliegenden Ergebnisse als spezifische Markerpeptide (Biomarker) zum diagnostischen Nachweis und zur Verlaufskontrolle von Entzündungen und Infektionen (insbesondere auch von systemischen Infektionen vom Sepsistyp) dienen. Wie die Bestimmung von Procalcitonin kann dabei die Bestimmung von Inflammin durch die Anwendung eines Verfahrens zur differentialdiagnostischen Früherkennung und zur Erkennung sowie zur Beurteilung des Schweregrads und zur therapiebegleitenden Verlaufsbeurteilung von Sepsis und Infektionen erfolgen, wobei man bei einem derartigen Verfahren in einer Probe einer biologischen Flüssigkeit oder eines Gewebes eines Patienten den Gehalt an Inflammin oder eines Teilpeptids davon bestimmt und aus der festgestellten Anwesenheit und/oder Menge des bestimmten Peptids auf das Vorliegen einer Entzündung, einer schweren Infektion oder einer Sepsis schließt und das erhaltene Ergebnis mit dem Schweregrad der Sepsis korreliert und die Behandlungsmöglichkeiten und/oder die Behandlungsaussichten abschätzt.

An Stelle der Bestimmung der Peptide Präinflammin oder Inflammin oder ihrer Bruchstücke oder ggf. posttranslational modifizierten Formen davon ist für diagnostische Zwecke auch die Bestimmung der zugehörigen mRNA möglich.

Inflammin oder seine Bruchstücke oder Fusionsprodukte oder die dafür kodierende DNA können auch in der präventiven Medizin oder Therapie verwendet werden. So können z.B. geeignete Inflammin-Bruchstücke zur in-vivo-Erzeugung von inflamminbindenden Antikörpern durch aktive Immunisierung nach an sich bekannten Techniken verwendet werden. Als Inflammin sollen dabei auch solche Moleküle anzusehen sein, die das vollständige Inflammin oder geeignete Teilseqenzen davon in posttranslational modifizierter Form, z.B. in glykosylierter oder phosphorylierter Form, oder auch in einer durch pharmazeutische Hilfsstoffe, z.B. Polyethylenglykolreste, substituierten Form enthalten. Ferner kann das Inflammin in oxidierter Form mit einer aus den beiden vorhandenen Cysteinresten gebildeten Disulfidbrücke oder in reduzierter Form vorliegen. Inflammin oder geeignete Teilsequenzen davon können auch in dem Sinne als Target für therapeutische Interventionen dienen, dass mittels geeigneter spezifischer Binder für Inflammin oder Teilpeptide davon Inflammin introkorporal inaktiviert wird oder ggf. auch extrakorporal im Sinne einer "Blutwäsche" bzw. Plasmapherese unter Verwendung geeigneter Immunadsorbentien eliminiert wird. Zur in-vivo-Inaktivierung von Inflammin kommen insbesondere spezifische Antikörper, insbesondere humanisierte monoklonale Antikörper, in Frage. Die therapeutische Beeinflussung der Entzündungskaskade kann aber auch unter Einsatz von Inflammin selbst oder von Inflamminagonisten oder -antagonisten erfolgen. Derartige therapeutische Interventionen werden insbesondere dann möglich, wenn weitere Erkenntnisse zur physiologischen Funktion des Inflammins abgesichert sind. So erscheint es derzeit nicht ausgeschlossen, dass Inflammin oder ein Teilpeptid davon im Entzündungsgeschehen eine wichtige Rolle spielt, möglicherweise als Mediator oder als hormoneller Regulator.

Nachfolgend wird die Auffindung und Identifizierung von Inflammin in näheren Einzelheiten geschildert, wobei auf das beigefügte Sequenzprotokoll und Bereiche davon bezug genommen wird. Die Figuren zeigen:
- Fig. 1: Ansichten von 2D-Elektrophoresegelen, die einen Vergleich der Spotmuster von cytoplasmatischen Leberzellprotein eines gesunden Pavians (A) mit den Leberzellproteinen eines Pavians 5h nach einer durch LPS-Verabreichung induzierten Sepsis (B) ermöglichen. Der Pfeil zeigt die Position des erfindungsgemäßen sepsisspezifischen Produkts Inflammin an, das in Darstellung (B) durch einen Kreis hervorgehoben ist;
- Fig. 2: das Massenspektrum des durch 2D-Gelelektrophorese identifizierten trypsinverdauten vollständigen Produkts Inflammin, und
- Fig. 3: die Ergebnisse einer Tandem-Elektrophorese eines selektierten Peptidfragments der Trypsinverdauung mit einem Ladung/Masse-Verhältnis von 660,80.

### 1. Infektionssimmulation durch Endotoxinverabreichung im Tiermodell (Paviane).

In Anlehnung an die mit Pavianen durchgeführten Versuche zur Stimulierung der Procalcitonin-Ausschüttung durch Endotoxininjektionen (vgl.H.Redl, et al., "Procalcitonin release patterns in a baboon model of trauma and sepsis: Relationship to cytokines and neopterin", Crit Care Med 2000, Vol.28. No.11, 3659-3663; H.Redl, et al., "Non-Human Primate Models of Sepsis", in: Sepsis 1998; 2:243-253) wurden Pavianen (männlich, ca. 2 Jahre alt, 27 bis 29 kg schwer) jeweils 100 µg LPS (Lipopolysaccharid aus Salmonella Typhimurium, Bezugsquelle: Sigma) pro kg Körpergewicht intravenös verabreicht. 5 bis 5,5 h nach der Injektion wurden die Tiere durch intravenöse Verabreichung von 10 ml Doletal getötet. Innerhalb von 60 min nach ihrem Exitus wurden sämtliche Organe/Gewebe präpariert und durch Einfrieren in flüssigem Stickstoff stabilisiert.

Bei der weiteren Verarbeitung wurden Proben der einzelnen tiefgefrorenen Gewebe (1g) unter Stickstoffkühlung mit 1,5 ml Puffer A (50mM Tris/HCl, pH 7,1, 100mM KCl, 20% Glycerol) versetzt und in einem Porzellanmörser zu einem Mehl pulverisiert (vgl. J.Klose, "Fractionated Extraction of Total Tissue Proteins from Mouse and Human for 2-D Electrophoresis", in: Methods in Molecular Biology, Vol.112: 2-D Proteome Analysis Protocols, Humana Press Inc., Totowa, NJ). Nach einer anschließenden 1-stündigen Zentrifugation bei 100.000 g und +4°C wurde der erhaltene Überstand gewonnen und bis zur weiteren Verarbeitung bei -80°C gelagert.

Unter Verwendung der auf diese Weise gewonnenen Gewebeextrakte wurde zuerst untersucht, in welchem der untersuchten Gewebe sich durch die Endotoxinverabreichung die höchsten Mengen des bekannten Sepsis-Biomarkers Procalcitonin erzeugen lassen. In dem ermittelten Gewebe mit der höchsten Procalcitoninbildung wurde dann mittels differentieller Proteomanalyse nach weiteren bisher nicht identifizierten proteinischen Produkten gesucht, die nur nach der Endotoxinverabreichung auftraten. Dazu wurden als Kontroll-Gewebeproben Gewebeproben unbehandelter Paviane verwendet, wobei die Tötung und Probengewinnung unter identischen Bedingungen erfolgte wie bei den behandelten Tieren.

### 2. Ermittlung von Paviangeweben mit der höchsten Procalcitoninbildung nach Endotoxininjektion.

Proben der einzelnen Gewebe wurden mit Hilfe eines Immunoluminometrischen Tests untersucht, der (in Anlehnung an den zur Bestimmung von humanem Procalcitonin entwickelten LU-MItest® PCT der Anmelderin) mit einem auf Polystyrolröhrchen immobilisierten Antikörper gegen Pavian-Calcitonin einerseits und einem mit einem Akridiniumester markierten monoklonalen Antikörper, der gegen den N-Terminus des Pavian-Procalcitonins gerichtet ist, arbeitet. Mit Hilfe dieses Tests wurden die Gehalte an Pavian-Procalcitonin in den einzelnen Proben nach Kalibrierung des Tests unter Verwendung von rekombinantem humanem Procalcitonin ermittelt.

Die Versuche ergaben, dass Lebergewebe die größte Procalcitoninmenge liefert. Für die Suche nach neuen sepsisspezifischen Biomarkern wurde daher mit den auf die eingangs beschriebene Weise gewonnenen Proteinextrakten aus Pavianleber gearbeitet.

### 3. Proteomanalyse unter Verwendung cytoplasmatischer Leberzellproteine von Pavianen.

Cytoplasmatische Leberzellproteinextrakte von einerseits gesunden Pavianen (Kontrolle) und andererseits Pavianen, denen LPS injiziert worden war, wurden im Rahmen einer Proteomanalyse verwendet. Bei der einleitenden analytischen 2D-Gelelektrophorese wurde Leberextrakt, 100 µg Protein enthaltend, auf 9M Harnstoff, 70 mM DTT, 2% Ampholyt pH 2-4 eingestellt und dann mittels analytischer 2D-Gelelektrophorese aufgetrennt, wie in J.Klose, et al., "Two-dimensional electrophoresis of proteins: An updated protocol and implications for a functional analysis of the genome", Electrophoresis 1995, 16, 1034-1059; beschrieben ist. Die Sichtbarmachung der Proteine im 2D-Elektrophoresegel erfolgte mittels Silverstaining (vgl. J.Heukeshoven, et al., "Improved silver staining procedure for fast staining in Phast-System Development Unit. I. Staining of sodium dodecyl gels", Electrophoresis 1988, 9, 28-32).

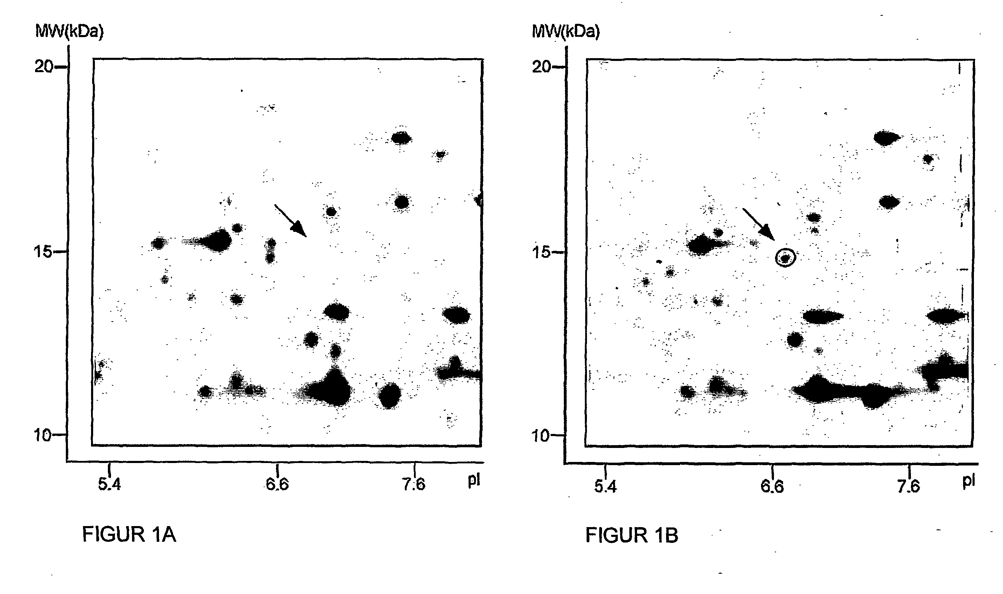

Zur Auswertung wurden die Proteinspotmuster der Proben unbehandelter Tieren mit den Proteinspotmustern verglichen, die aus Lebergewebeproben behandelter Tiere resultierten. Substanzen, die bei keiner Kontrollprobe, aber bei allen behandelten Tieren zusätzlich auftraten, wurden für weitere analytische Untersuchungen selektiert. Fig. 1 zeigt einen Vergleich der 2D-Elektrophoresegele für eine Kontrollprobe (A) und eine Probe eines behandelten Tieres (B), wobei der zusätzliche Proteinspot in (B) Inflammin entspricht, dessen Position durch einen Pfeil und einen Kreis hervorgehoben ist.

Die im Proteinspotmuster der analytischen 2D-Gelelektrophorese identifizierten neuen spezifischen Proteine wurden dann anschließend mittels präparativer 2D-Gelelektrophorese unter Einsatz von 350 µg Protein präpariert (vgl. wiederum (10). Bei der präparativen 2D-Gelelektrophorese erfolgte die Färbung mittels Coomassie Brilliant Blue G250 (vgl. V.Neuhoff, et al., "Improved staining of proteins in polyacrylamide gels including isoelectric focusing gels with clear background at nanogram sensitivity using Coomassie Brilliant Blue G-250 and R-250", Electrophoresis 1988, 9, 255-262).

Die für die weitere Analyse vorselektierten Proteinspots wurden aus dem Gel ausgeschnitten, unter Anwendung der Methode, die in A.Otto, et al., "Identification of human myocardial proteins separated by two-dimensional electrophoresis using an effective sample preparation for mass spectrometry", Electrophoresis 1996, 17, 1643-1650; beschrieben ist, trypsinverdaut und anschließend massenspektroskopisch analysiert und zwar unter Anwendung massenspektrometrischer Untersuchungen, wie sie z.B. in G.Neubauer, et al., "Mass spectrometry and EST-database searching allows characterization of the.multi-protein spliceosome complex", in: nature genetics vol. 20, 1998, 46-50; J.Lingner, et al., "Reverse Transcriptase Motifs in the Catalytic Subunit of Telomerase", in: Science, Vol.276, 1997, 561-567; M.Mann, et al., "Use of mass spectrometry-derived data to annotate nucleotide and protein sequence databases", in: TRENDS in Biochemical Sciences, Vol.26, 1, 2001, 54-61; beschrieben und diskutiert werden. Dabei wurden die trypsinverdauten Proben nach einer ESI (ElectroSprayIonisierung) einer Tandem-Massenspektrometrie unterzogen. Es wurde ein Q-TOF-Massenspektrometer mit einer sog. nanoflow-Z-Spray-Ionenquelle der Firma Micromass, UK, verwendet. Dabei wurde entsprechend der Arbeitsanleitung des Geräteherstellers gearbeitet.

### 4. Identifizierung von Inflammin

Wie in den Figuren 1(A) und 1(B) gezeigt ist, findet sich in Leberzellextrakten von Pavianen, denen eine LPS-Injektion verabreicht worden war, u.a. ein neues Protein, für das aufgrund der Gelektrophoresedaten im Vergleich mit Markersubstanzen mit bekanntem Molekulargewicht ein Molekulargewicht von ca. 15.000 ± 700 Dalton abgeschätzt wurde, während aus der relativen Position des Proteins aus der ersten Dimension ein isoelektrischer Punkt von ca. 6,5 bis 7 abgeschätzt wurde.

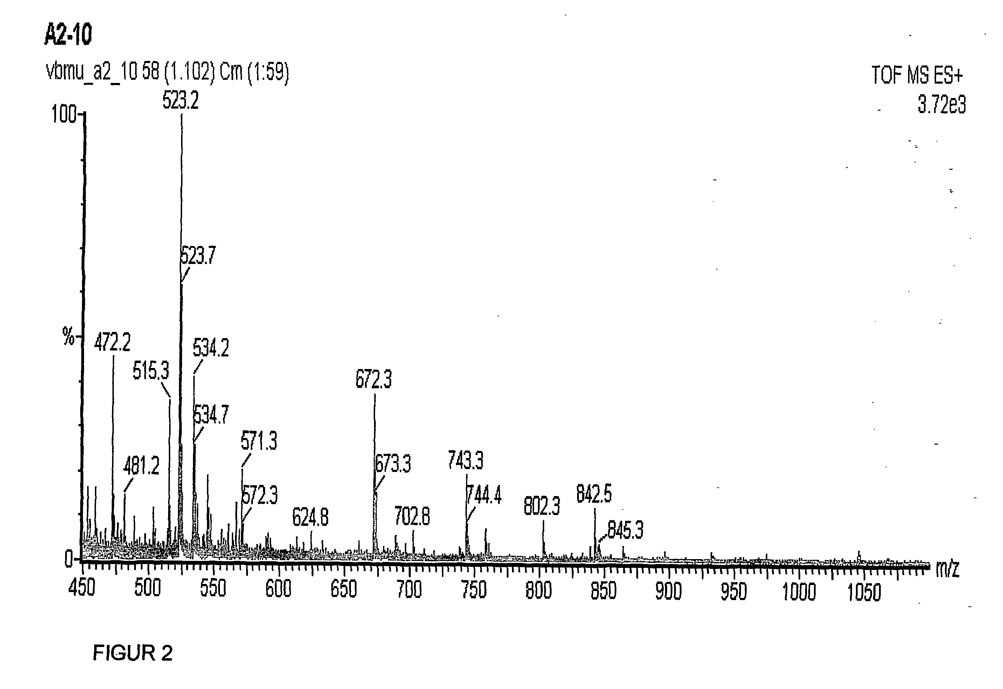

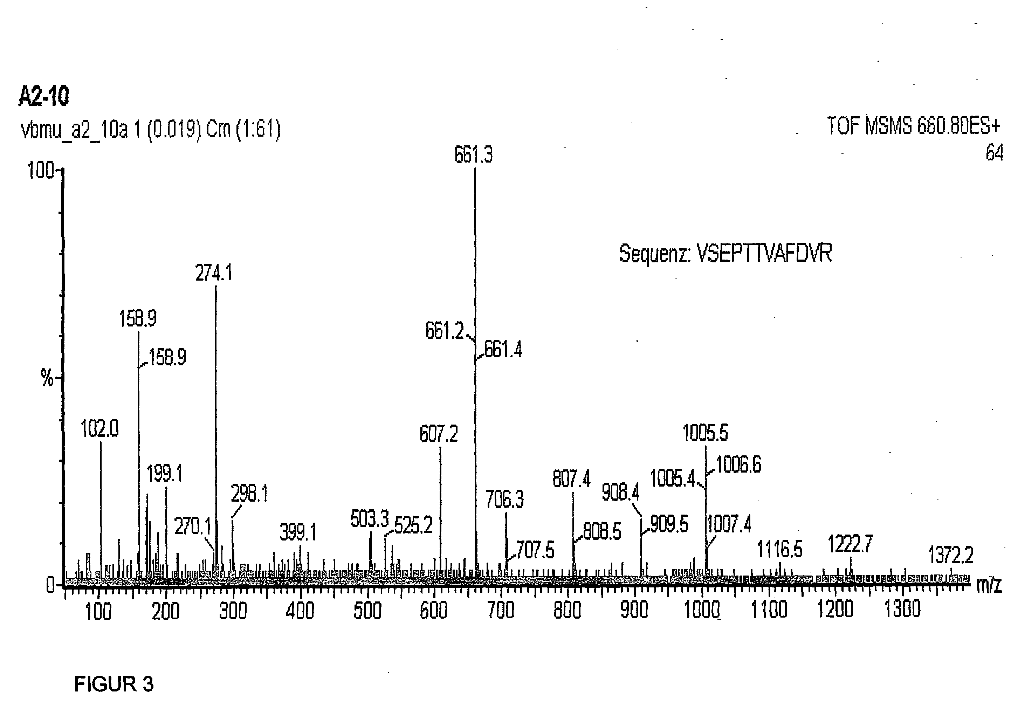

Dieses Protein wurde wie oben massenspektrometrisch analysiert, wobei Fig. 2 das Massenspektrum des gesamten trypsinverdauten Proteins zeigt, während Fig. 3 das Spektrum des durch Tandem-Massenspektroskopie selektierten Fragments mit einer Masse/Z von 660,8 zeigt. Das für dieses Fragment erhaltene Massenspektrum konnte auf sich bekannte Weise rechnerisch der Teilsequenz SEQ ID NO:1 zugeordnet werden. Diese Teilsequenz wurde dann mit Proteinsequenzen verglichen, die in Sequenzdatenbanken verfügbar waren (OWL-Datenbank http://www.matrixscience.com; bzw. http://www.ch.embnet.org). In der redundanten Datenbank NCBI nr.2.13.2001 wurden zwei Sequenzen ermittelt, in denen sich das Fragment gemäß SEQ ID NO:1 zu 100% wiederfindet. Die beiden Sequenzsegmente können einem hypothetischen Protein R33729_1 aus dem Human-Genom-Projekt zugeordnet werden, wobei die eine Sequenz (SEQ ID NO:2) eine Teilsequenz mit 152 Aminosäuren (AAC33800; DBSOURCE locus AC005594; Accession AC005594.1) mit der Bezeichnung "partial CDS" ist, während die zweite Teilsequenz (SEQ ID NO:3) eine Sequenz mit 170 Aminosäuren (Locus CAB96947; DBSOURCE embl locus IR1875335; accession AL365373.1) mit der Bezeichnung "hypothetical protein" ist. Beide Sequenzen sind Teile eines hypothetischen humanen Proteins, dessen codierende Sequenz sich auf dem Chromosom 19 befindet. Die erste Teilsequenz ("partial CDS") ist die im Sequenzprotokoll wiedergegebene Sequenz SEQ ID NO:2, während die zweite Teilsequenz die Sequenz gemäß Sequenzprotokoll SEQ ID NO:3 ist. Die Teilsequenz SEQ ID NO:2 enthält das Startcodon (Positionen 1-3) des gesamten hypothetischen Proteins, während die zweite Teilsequenz SEQ ID NO:3 zusätzliche Aminosäuren vor einem Stopcodon (Positionen 145-170) enthält, so dass durch das Zusammensetzen beider Sequenzen das gesamte durch Ribosomen produzierte Protein SEQ ID NO:5 dargestellt werden kann. Die einzige Abweichung innerhalb beider Sequenzen SEQ ID NO:2 und SEQ ID NO:3 ist ein Pentapeptid in der Position 59 bis 63 der Sequenz SEQ ID NO:2, das in der zweiten Teilsequenz kein Gegenstück hat. SEQ ID NO:2 basiert auf einer theoretischen Exon/Intron-Vorhersage an genomischer DNA, während SEQ ID NO:3 sich von einer cDNA ableitet. Vier ähnliche cDNAs aus Maus (BAB25579, BAB25931, BAB23155, BAB31038) enthalten wie die menschliche cDNA keine entsprechende Pentapeptid-Sequenz. Somit ist es eher wahrscheinlich, dass das gefundene humane Polypeptid diese Pentapeptid-Sequent nicht enthält. Selbst wenn man vom Fehlen des genannten Pentapeptids ausgeht, liegt aber die theoretische Molmasse von SEQ ID NO:5 (ca. 19kDa) über dem tatsächlich für den neuen Proteinspot gefundenen Wert (15 ± 0,7 kDa). Die Überprüfung von SEQ ID NO:5 in einem Vorhersageprogramm (http://www.cbs.dtu.dk/services/SignalP/) für N-terminale Signalsequenzen, die verantwortlich für die extrazelluläre Lokalisation von Polypeptiden sein können, ergab, dass mit großer Wahrscheinlichkeit die ersten 31 Aminosäuren von SEQ ID NO:5 eine Signalsequenz darstellen, die bei der Translokation des naszierenden Polypeptids über die Membran des Endoplasmatischen Retikulums von der Signalpeptidase abgespalten werden. Eine solche Verkürzung ergäbe eine gute Übereinstimmung mit der bei der Gelelektrophorese tatsächlich beobachteten Molmasse. Eine gleiche Analyse mit den o.g. ähnlichen Maus-Sequenzen liefert eine analoge Vorhersage. Ein experimentelles Beweisanzeichen für diese Verkürzung stellt das Auffinden des Peptids SEQ ID NO:1 dar. Dieses wurde massenspektrometrisch nach Behandlung des neuen Proteinspots aus der Gelelektrophorese mit Trypsin identifiziert. Dabei ist von Bedeutung, dass in SEQ ID NO:1 in der Position N-terminal von SEQ ID NO:5 keine basische Aminosäure vorliegt, wie sie für eine Trypsinverdauung typisch wäre. Dagegen entspricht der gefundene Schnitt genau einem Schnitt, wie er unter der Einwirkung der Signalpeptidase erwartet würde. Das Protein (Peptid) SEQ ID NO:5 soll daher als Präinflammin bezeichnet werden, während für das putative reife Inflammin die SEQ ID NO:4 vorgeschlagen wird. SEQ ID NO:4 unterscheidet sich dadurch von SEQ ID NO:5, dass dessen Positionen 1-31 sowie 59-63 fehlen.

Die bekannte vollständige Sequenz gestattet es, für diagnostische Zwecke das gesamte Peptid oder beliebige Teilsequenzen (Fragmente) davon gezielt herzustellen, und zwar unter Anwendung bekannter synthetischer oder gentechnologischer Verfahren zur Herstellung von Peptiden. Derartige Peptide können dann, beispielsweise in Analogie zu der in P.P.Ghillani, et al., "Monoclonal antipeptide antibodies as tools to dissect closely related gene products", The Journal of Immunology, vol. 141, No.9, 1988, 3156-3163; und P.P.Ghillani, et al., "Identification and Measurement of Calcitonin Precursors in Serum of Patients with Malignant Diseases", Cancer research, vol.49, No.23, 1989, 6845-6851; beschriebenen Arbeitsweise zur Schaffung von geeigneten Antikörpern, insbesondere monoklonalen Antikörpern dienen, die ihrerseits die Schaffung von Assays für die immundiagnostische Bestimmung des Inflammins oder ausgewählter Teilpeptide davon ermöglichen.

Auf die skizzierte bekannte Weise erhältliche monoklonale Antikörper können auch, insbesondere nach einer an sich bekannten Humanisierung, der Entwicklung von neuen Therapeutika dienen (vgl. die in K.Garber, "Protein C may be sepsis solution", Nature Biotechnology, Vol.18, 2000, 917-918; zusammengefassten therapeutischen Ansätze). Ferner ist eine in-vivo-Neutralisierung des Inflammins auch durch Blockade der Expression des Inflammingens möglich.

## Patentansprüche

1. Verwendung eines Peptids gemäß SEQ ID NO:4 oder SEQ ID NO:5 als Markerpeptid in in vitro Verfahren zum diagnostischen Nachweis und zur Verlaufskontrolle von Entzündungen und Infektionen.

2. Verwendung nach Anspruch 1, wobei eine Teilsequenz eines Peptids gemäß SEQ ID NO:4 oder SEQ ID NO:5 bestimmt wird, die die Aminosäuresequenz gemäß SEQ ID NO:1 aufweist.

3. Verwendung nach Anspruch 1 oder 2 zum Nachweis durch indirekten (PCR-) oder direkten Nachweis der zugehörigen mRNA.

4. Verwendung nach Anspruch 1, 2 oder 3 im Rahmen der differentialdiagnostischen Früherkennung und Erkennung, der Beurteilung des Schweregrads und der therapiebegleitenden Verlaufsbeurteilung von Sepsis und schweren Infektionen durch Bestimmung des genannten Peptids oder eines Fragments davon oder der mRNA für dieses Peptid in einer biologischen Flüssigkeit oder einer Gewebeprobe eines Patienten.

## Claims

1. Use of a peptide according to SEQ ID NO:4 or SEQ ID NO:5 as a marker peptide in in vitro methods for diagnostic detection or for monitoring the course of inflammations and infections.

2. Use according to Claim 1, wherein a partial sequence of a peptide according to SEQ ID NO:4 or SEQ ID NO:5 is determined, which has the amino acid sequence according to SEQ ID NO:1.

3. Use according to Claim 1 or Claim 2 for the detection by indirect (PCR) or direct detection of the corresponding mRNA.

4. Use according to Claim 1, 2 or 3 in the differential early diagnosis and diagnosis, the assessment of the severity and the therapy-accompanying assessment of the course of sepsis and severe infections by determination of said peptide or of a fragment thereof or of the mRNA for this peptide in a biological fluid or a tissue sample of a patient.

## Revendications

1. Utilisation d'un peptide de SEQ ID NO:4 ou SEQ ID NO:5 comme peptide marqueur dans des procédés in vitro de détection, de diagnostic et de contrôle de l'évolution d'inflammations et d'infections.

2. Utilisation selon la revendication 1, dans laquelle on détermine une séquence partielle d'un peptide de SEQ ID NO:4 ou SEQ ID NO:5, qui présente la séquence d'acides aminés de SEQ ID NO:1.

3. Utilisation selon les revendications 1 ou 2 pour la détection par détection indirecte (détection PCR) ou directe de l'ARNm associé.

4. Utilisation selon les revendications 1, 2 ou 3 dans le cadre de la détection précoce par diagnostic différentiel et de la détection par diagnostic différentiel de septicémies et d'infections graves, de l'évaluation de leur degré de gravité et de l'évaluation de leur évolution en accompagnement de la thérapie, par la détermination dudit peptide, d'un de ses fragments ou de l'ARNm de ce peptide dans un liquide biologique ou dans un échantillon de tissu d'un patient.


### SEQUENZPROTOKOLL

<110> B.R.A.H.M.S Aktiengesellschaft
<120> Entzündungsspezifische Peptide und deren Verwendungen
<130> 3473 PCT
<140>
   <141>
<160> 5
<170> PatentIn Ver. 2.1
<210> 1
   <211> 12
   <212> PRT
   <213> Primat (Pavian)
<400> 1
   Val Ser Glu Pro Thr Thr Val Ala Phe Asp Val Arg
    1               5                  10
<210> 2
   <211> 152
   <212> PRT
   <213> Homo sapiens
<400> 2
   Met Ala Ala Pro Ser Gly Gly Trp Asn Gly Val Gly Ala Ser Leu Trp
    1               5                  10                  15
   Ala Ala Leu Leu Leu Gly Ala Val Ala Leu Arg Pro Ala Glu Ala Val
               20                  25                  30
   Ser Glu Pro Thr Thr Val Ala Phe Asp Val Arg Pro Gly Gly Val Val
           35                  40                  45
   His Ser Phe Ser His Asn Val Gly Pro Gly Val Arg Ala Thr Ala Asp
       50                  55                  60
   Lys Tyr Thr Cys Met Phe Thr Tyr Ala Ser Gln Gly Gly Thr Asn Glu
   65                  70                  75                  80
   Gln Trp Gln Met Ser Leu Gly Thr Ser Glu Asp His Gln His Phe Thr
                   85                  90                  95
   Cys Thr Ile Trp Arg Pro Gln Gly Lys Ser Tyr Leu Tyr Phe Thr Gln
               100                 105                 110
   Phe Lys Ala Glu Val Arg Gly Ala Glu Ile Glu Tyr Ala Met Ala Tyr
           115                 120                 125
   Ser Lys Ala Ala Phe Glu Arg Glu Ser Asp Val Pro Leu Lys Thr Glu
       130                 135                 140
   Glu Phe Glu Val Thr Lys Thr Ala
   145                 150
<210> 3
   <211> 170
   <212> PRT
   <213> Homo sapiens
<400> 3
   Pro Ser Gly Gly Trp Asn Gly Val Gly Ala Ser Leu Trp Ala Ala Leu
   1 5 10 15
   Leu Leu Gly Ala Val Ala Leu Arg Pro Ala Glu Ala Val Ser Glu Pro
   20 25 30
   Thr Thr Val Ala Phe Asp Val Arg Pro Gly Gly Val Val His Ser Phe
   35 40 45
   Ser His Asn Val Gly Pro Gly Asp Lys Tyr Thr Cys Met Phe Thr Tyr
   50 55 60
   Ala Ser Gln Gly Gly Thr Asn Glu Gln Trp Gln Met Ser Leu Gly Thr
   65 70 75 80
   Ser Glu Asp His Gln His Phe Thr Cys Thr Ile Trp Arg Pro Gln Gly
   85 90 95
   Lys Ser Tyr Leu Tyr Phe Thr Gln Phe Lys Ala Glu Val Arg Gly Ala
   100 105 110
   Glu Ile Glu Tyr Ala Met Ala Tyr Ser Lys Ala Ala Phe Glu Arg Glu
   115 120 125
   Ser Asp Val Pro Leu Phe Thr Glu Glu Phe Glu Val Thr Lys Thr Ala
   130 135 140
   Val Ala His Arg Pro Gly Ala Phe Lys Ala Glu Leu Ser Lys Leu Val
   145 150 155 160
   Ile Val Ala Lys Ala Ser Arg Thr Glu Leu
   165 170
<210> 4
   <211> 142
   <212> PRT
   <213> Homo sapiens
<400> 4
   Val Ser Glu Pro Thr Thr Val Ala Phe Asp Val Arg Pro Gly Gly Val
   1 5 10 15
   Val His Ser Phe Ser His Asn Val Gly Pro Gly Asp Lys Tyr Thr Cys
   20 25 30
   Met Phe Thr Tyr Ala Ser Gln Gly Gly Thr Asn Glu Gln Trp Gln Met
   35 40 45
   Ser Leu Gly Thr Ser Glu Asp His Gln His Phe Thr Cys Thr Ile Trp
   50 55 60
   Arg Pro Gln Gly Lys Ser Tyr Leu Tyr Phe Thr Gln Phe Lys Ala Glu
   65 70 75 80
   Val Arg Gly Ala Glu Ile Glu Tyr Ala Met Ala Tyr Ser Lys Ala Ala
   85 90 95
   Phe Glu Arg Glu Ser Asp Val Pro Leu Lys Thr Glu Glu Phe Glu Val
   100 105 110
   Thr Lys Thr Ala Val Ala His Arg Pro Gly Ala Phe Lys Ala Glu Leu
   115 120 125
   Ser Lys Leu Val Ile Val Ala Lys Ala Ser Arg Thr Glu Leu
   130 135 140
<210> 5
   <211> 178
   <212> PRT
   <213> Homo sapiens
<400> 5
   Met Ala Ala Pro Ser Gly Gly Trp Asn Gly Val Gly Ala Ser Leu Trp
   1 5 10 15
   Ala Ala Leu Leu Leu Gly Ala Val Ala Leu Arg Pro Ala Glu Ala Val
   20 25 30
   Ser Glu Pro Thr Thr Val Ala Phe Asp Val Arg Pro Gly Gly Val Val
   35 40 45
   His Ser Phe Ser His Asn Val Gly Pro Gly Val Arg Ala Thr Ala Asp
   50 55 60
   Lys Tyr Thr Cys Met Phe Thr Tyr Ala Ser Gln Gly Gly Thr Asn Glu
   65 70 75 80
   Gln Trp Gln Met Ser Leu Gly Thr Ser Glu Asp His Gln His Phe Thr
   85 90 95
   Cys Thr Ile Trp Arg Pro Gln Gly Lys Ser Tyr Leu Tyr Phe Thr Gln
   100 105 110
   Phe Lys Ala Glu Val Arg Gly Ala Glu Ile Glu Tyr Ala Met Ala Tyr
   115 120 125
   Ser Lys Ala Ala Phe Glu Arg Glu Ser Asp Val Pro Leu Phe Thr Glu
   130 135 140
   Glu Phe Glu Val Thr Lys Thr Ala Val Ala His Arg Pro Gly Ala Phe
   145 150 155 160
   Lys Ala Glu Leu Ser Lys Leu Val Ile Val Ala Lys Ala Ser Arg Thr
   165 170 175
   Glu Leu